# EUROPEAN PATENT SPECIFICATION

(11) **EP 0 579 598 B1**
(45) Date of publication and mention of the grant of the patent: **03.01.1996**
(21) Application number: 91907658.8
(22) Date of filing: 12.04.1991
(51) Int. Cl.: H01F 7/02, G01R 33/38

(54) **PERMANENT MAGNET ASSEMBLY**
PERMANENTMAGNETANORDNUNG
ENSEMBLE A AIMANTS PERMANENTS

(43) Date of publication of application: 26.01.1994
(73) Proprietor: ELBIT-ATI, LTD., Oak Park, IL 60304 (US); TOMLINSON, Kerry John, East Sussex BN3 6LL (GB)
(72) Inventor: RAPOPORT, Uri, Oak Park, IL 60302 (US)
(74) Representative: Tomlinson, Kerry John
(86) International application number: EP9100716
(87) International publication number: WO9218993

(56) References cited:
- US-A- 3 611 223
- US-A- 4 675 609
- US-A- 4 692 732
- US-A- 4 707 663
- US-A- 4 998 976

## Description

This invention relates to a permanent magnet assembly, and is especially useful in the field of magnetic resonance imaging and spectroscopy. Optimum performance of magnetic resonance devices requires precise alignment of pole pieces of opposite magnetic polarity. It is also important to minimize flux leakage at the interface between certain components.

US-A-4,998,976 discloses a permanent magnet assembly used for creating a uniform magnetic field across a portion of an air gap. This includes a principal magnet, a pole piece and a set of auxiliary magnets extending about the periphery of the pole piece. Two such magnet groupings are positioned within a test chamber, separated by spacers.

It would be desirable to have an arrangement for accurately aligning pole pieces and for minimizing flux leakage at certain interfaces.

It is a principal object of the invention to provide a permanent magnet assembly having an improved arrangement for aligning opposite pole pieces on opposite sides of a central spacer.

It is another object of the invention to provide a permanent magnet assembly having an improved arrangement for minimizing flux leakage at a peripheral interface between a pole piece and side permanent magnets.

According to the invention, there is provided a permanent magnet assembly for providing a magnetic field across a gap and including opposed magnet members having opposite magnetic poles facing one another across a gap, and spacer means of non-ferromagnetic material positioned between said magnet members for maintaining same in spaced-apart relationship, said magnet members having facing end portions adjacent said spacer means, characterized by cooperating alignment means between said magnet members and said spacer means for aligning said magnet members along a substantially common longitudinal axis; wherein said alignment means comprises opposite shallow recesses in said spacer means closely receiving said facing end portions of said magnet members.

In a feature of the invention, main permanent magnets abut the pole pieces or magnet members on the opposite sides thereof from the central spacer means. The main permanent magnets are slightly larger than the pole pieces, and outwardly overlap a peripheral interface between the pole pieces and side permanent magnets assembled therearound. This overlap minimizes flux leakage at the peripheral interface between a pole piece and side magnets.

In another feature electro-magnetic shunt coils on the central spacer means have lead wires extending through tubes of ferromagnetic material that are slidably received in longitudinal grooves in a ferromagnetic housing.

In another feature, projection guide means on the central spacer means is closely received in a longitudinal groove in the housing for angularly aligning a transverse hole in the central spacer means with a transverse hole in the housing.

A permanent magnet assembly in accordance with the present application preferably has a one-piece housing, not counting the end caps. However, the housing can be longitudinally or transversely split. In the preferred arrangement, the housing is cylindrical. However, certain features of the present application are also advantageous in housings of other shapes, such as a housing having a square cross-sectional shape.

An embodiment of the invention will now be described by way of example only and with reference to the accompanying drawings.
Figure 1 is an exploded perspective illustration of a permanent magnet assembly constructed in accordance with the present application;
Figure 2 is a cross-sectional elevational view of the assembly of Figure 1;
Figure 3 is a plan view of a central spacer used in the assembly;
Figure 4 is a partial top elevational view of one end portion of a ferromagnetic housing showing a longitudinal groove therein;
Figure 5 is a side elevational view of a ferromagnetic tube receivable in the groove of Figure 4;
Figure 6 is a cross-sectional elevational view showing the tube of Figure 5 attached to a projection on the central spacer of Figure 3, and with lead wires from electro-magnetic shunt coils extending from the spacer through the tube and
Figure 7 is a detail view of the central part of Fig. 2 taken on the line VII-VII of Fig. 3.

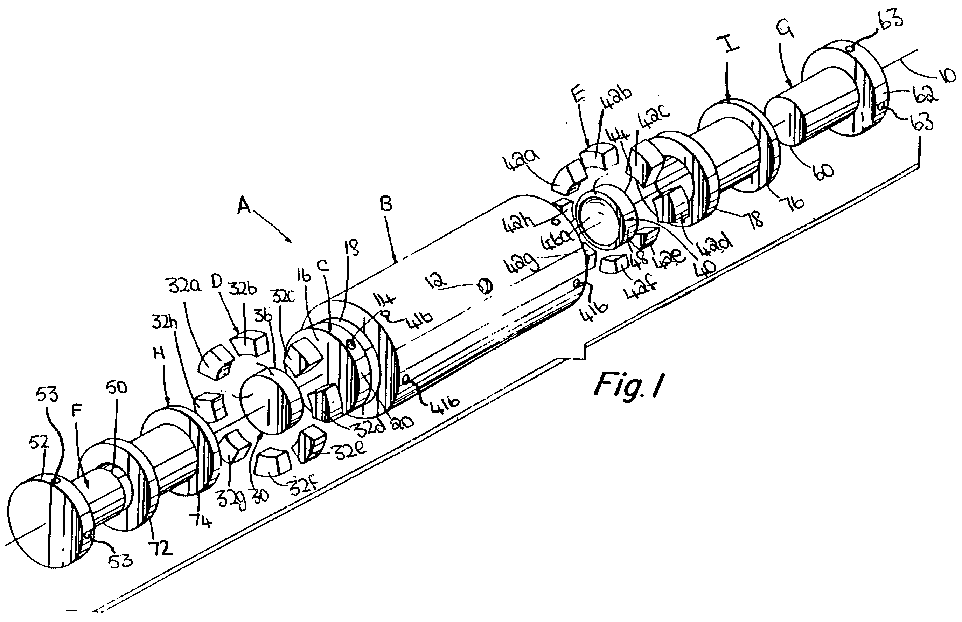

Referring now to the drawings, wherein the showings are for purposes of illustrating certain preferred embodiments of the invention only and not for purposes of limiting same, Figure 1 is an exploded perspective illustration of a permanent magnet assembly that includes a hollow cylindrical housing B of ferromagnetic material and having a longitudinal axis 10. The peripheral wall of housing B has a pair of holes therethrough located 180° apart, and only one such hole is indicated at 12 in Figure 1. The pair of holes have centers lying on a common axis extending perpendicular to and intersecting the longitudinal axis of housing B. The centers of the holes are also located centrally between the opposite ends of housing B.

A cylindrical spacer disc C of nonmagnetic material is a close sliding fit within housing B, and has a hole 14 therethrough of the same diameter as hole 12. The axis of hole 14 in spacer disc C extends perpendicular to and intersects longitudinal axis 10 of disc C. Spacer disc C has substantially plane and parallel opposite faces 16, 18 and a cylindrical outer periphery 20. The axis of hole 14 is centrally located between faces 16, 18.

Each of a pair of permanent magnet subassemblies D, E includes a pole piece 30, 40 of ferromagnetic material, and a plurality of side permanent magnets 32a-h, 42a-h. Pole piece 30 has opposite faces 34, 36 and a cylindrical outer periphery 38. Pole piece 40 has opposite faces 44, 46 and a cylindrical outer periphery 48. At least faces 34, 44 are substantially flat and lie in planes extending substantially perpendicular to longitudinal axis 10.

Side permanent magnet means is shown in the form of eight arcuate side permanent magnets 32a-h, each extending over an arc of approximately 45°, so they extend substantially continuously around the periphery of pole piece 30. The inner surfaces of side magnets 32a-h are curved to lie on the periphery of a common cylinder with outer peripheral surface 38 of pole piece 30. Thus, when side magnets 32a-h are positioned against outer periphery 38 of pole piece 30, such side magnets extend completely around pole piece 30 in surrounding relationship thereto. In addition, the outer peripheral surfaces of side magnets 32a-h then lie on the periphery of a cylinder and have a very close sliding fit within housing B. The fit is such that the outer peripheral surfaces of the side magnets actually engage the inner peripheral surface of housing B.

Each side magnet 32a-h has a longitudinal thickness such that when the axially inwardly facing surface thereof is positioned against face 16 of spacer disc C, the opposite outwardly facing surface thereof is flush with outer face 34 of pole piece 30. There are also eight arcuate side permanent magnets 42a-h, and the relationship between them and pole piece 40 is the same as described with respect to the relationship between side magnets 32a-h and pole piece 30. Thus, when the magnet is assembled, the outwardly facing surfaces of side magnets 32a-h are substantially flush with outer face 34 of pole piece 30, and the outwardly facing surfaces of side magnets 42a-h are substantially flush with outer face 44 of pole piece 40.

The side permanent magnets are magnetized generally radially of the longitudinal axis of their respective pole pieces. All of the side permanent magnets in one permanent magnet subassembly D or E are magnetized to have north magnetic poles on their inner peripheral surfaces and south magnetic poles on their outer peripheral surfaces, while all of the side permanent magnets in the other permanent magnet subassembly are magnetized to have south magnetic poles on their inner peripheral surfaces and north magnetic poles on their outer peripheral surfaces. Therefore, one of pole pieces 30 or 40 is a north magnetic pole, while the other pole piece is a south magnetic pole, so that the pole pieces are of opposite magnetic polarity.

The side permanent magnet means can take many other forms. For example, the number of individual side magnets can be greater or smaller than eight, including one.

Cylindrical main permanent magnets F, G have a diameter that is slightly larger than the diameter of pole pieces 30, 40 and are axially concentric therewith. Main permanent magnet F has one flat end 50 positionable in abutting relationship with face 34 of pole piece 30, and an opposite flat end attached to a cylindrical housing cover 52 of ferromagnetic material. Cylindrical main permanent magnet G has a flat end 60 positionable against face 44 of pole piece 40, and an opposite flat face attached to cylindrical cover 62 of ferromagnetic material. Covers 52, 62 are a close sliding fit within housing B. Ends 50, 60 of main magnets F, G also overlie and abut portions of the outwardly facing surfaces of side magnets 32a-h and 42a-h that are located closely adjacent faces 34, 44 of pole pieces 30, 40.

Covers 52, 62 have a plurality of circumferentially-spaced threaded bores 53, 63 alignable with radial holes 416 in housing B. Fasteners are receivable through holes 416 within bores 53, 63 for securing the covers to the housing.

Spool-like sleeve members H, I of nonmagnetic material have axial cylindrical holes therethrough for receiving permanent magnets F, G with a close sliding fit. The axial length of each sleeve member H, I is substantially the same as the axial length of a main permanent magnet F, G. Sleeve member H has cylindrical flanges 72, 74 that are a close sliding fit within housing B. Sleeve member I has cylindrical flanges 76, 78 that are a close sliding fit within housing B. When all of the components are positioned in end-to-end abutting relationship within housing B, the outer surfaces of covers 52, 62 are substantially flush with the end surfaces of housing B.

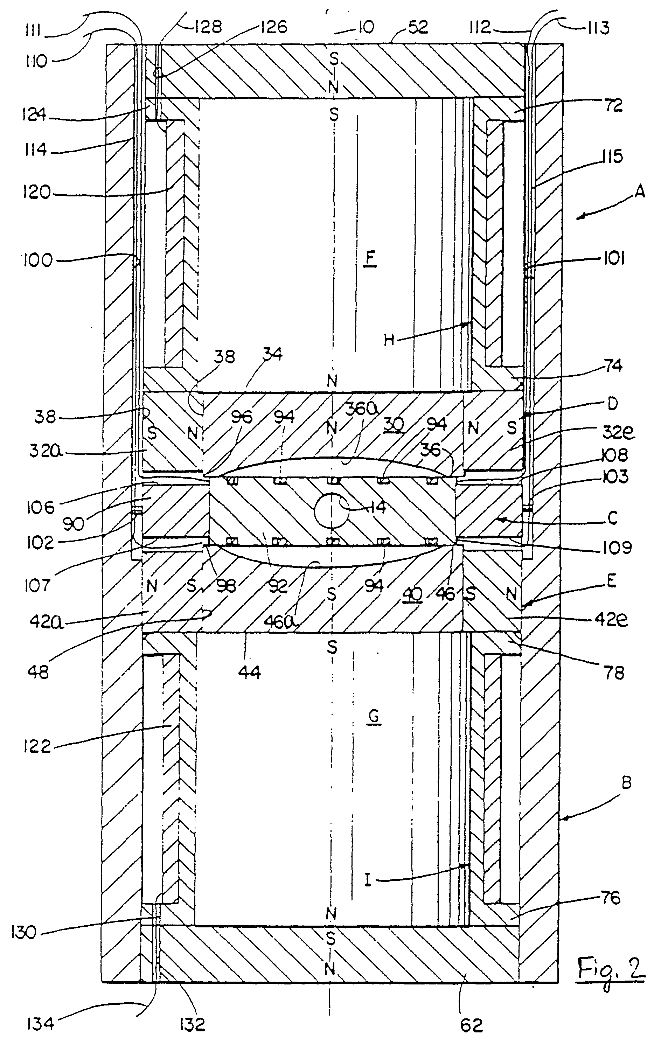

Figure 2 shows the components of Figure 1 in assembled relationship. The magnetic polarity of the magnetized components is indicated by N and S. Spacer disc C includes an outer ring portion 90 of nonmagnetic material having a central circular hole therethrough receiving a central disc 92 of dielectric material. Hole 14 extends through both ring 90 and disc 92, and that portion of the hole located in the area of longitudinal axis 10 defines a test zone in which materials to be tested are positioned. Recesses in the opposite faces of dielectric central disc 92 receive windings 94 of electro-magnetic shunt coils for selectively adjusting the magnetic field in the test zone within hole 14 between poles 30, 40. Cooperating alignment means is provided between spacer C and pole pieces 30, 40 for aligning such pole pieces along a common longitudinal axis. The alignment means may comprise suitably shaped cooperating projections and recesses, a most preferred form of which is shown in the drawing wherein central circular recesses 96, 98 on the opposite surfaces of ring portion 90 receive end portions of pole pieces 30, 40 with a close sliding fit to center same with respect to spacer disc C and to one another. The opposite outer surfaces of disc 92 are substantially flush with the bottoms of recesses 96, 98.

As shown in Figure 2, the axial thickness of each pole piece 30, 40 is greater than such thickness of its associated side magnets 32a-h, 42a-h by an amount equal to the depth of a recess 96 or 98 in ring portion 90. Recesses 96, 98 are preferably very shallow so that the axial thickness of the side magnets can be maximized. The depth of recesses 96, 98 is preferably just sufficient to receive end portions of pole pieces 30, 40 therein for alignment purposes. The recesses help to align the pole pieces along a common longitudinal axis coincidental with longitudinal axis 10, and also help to ensure that flat facing surfaces 36, 46 are parallel.

Main magnets F, G extend outwardly slightly beyond and overlap the circular abutting lines between the outer peripheries 38, 48 of pole pieces 30, 40 and the inner peripheral surfaces of side magnets 32a-h, 42a-h in order to minimize outward flux leakage. If the main magnets have a diameter that is the same or slightly smaller than the pole pieces, outward flux leakage is possible at the interface between the outer periphery of a pole piece and the inner periphery of the side magnets. Overlapping such interface with the main magnets inhibits such flux leakage because the force fields of the main magnets oppose such leakage.

Faces 36, 46 of pole pieces 30, 40 have centrally located curved depressions 36a, 46a therein symmetrical about longitudinal axis 10. Although depressions could extend all the way to outer peripheries 38, 48 of pole pieces 30, 40, they are preferably spaced inwardly therefrom to provide flat bearing areas engaging the bottoms of recesses 96, 98 and portions of the outer surfaces of disc 92. Each depression 36a, 46a preferably occupies substantially more than one-half the total area of each face 36, 46 on pole pieces 30, 40.

In the most preferred arrangement, the entire surface area of depressions 36a, 46a is everywhere curved and non-perpendicular to longitudinal axis 10. In general, the curvature of the depressions will vary from the outer periphery thereof to the center thereof. That is, the curvature is such that it is not a regular geometric surface, such as the surface of a sphere or parabola, or a surface that can be generated by rotating a regular geometric curve, such as a parabola, about its major or minor axis. The best curvature can be determined by computer modeling, by trial and error, or empirically.

Magnetic flux enters and leaves magnet surfaces perpendicular to such surfaces. When surfaces 36, 46 of pole pieces 30, 40 are completely flat and perpendicular to longitudinal axis 10, the magnetic flux enters and leaves such surfaces perpendicular thereto and parallel to axis 10. However, in the space between the pole pieces, the flux tends to bow outwardly away from axis 10, and this reduces the strength and uniformity of the magnetic field in the test zone.

Curved depressions 36a, 46a are designed to impart an inward bowing effect on the flux lines in the space between the pole pieces. The curvature is preferably such that the inward bowing effect thereof is precisely matched by the outward bowing forces so that the flux lines in the test zone are actually substantially parallel to longitudinal axis 10. This enhances the strength and uniformity of the magnetic field in the test zone.

Housing B has a pair of longitudinal grooves 100, 101 in the inner surface thereof located 180° apart from one another, and spaced 90° from the central axis of housing hole 12 of Figure 1. Pins 102, 103 fixed to spacer disc C extend radially outwardly therefrom at locations spaced 90° from the center of hole 14. Pins 102, 103 are receivable in grooves 100, 101 for aligning hole 14 with hole 12 when spacer disc C is moved into housing B. Ring portion 90 of spacer disc C has radially extending grooves 106-109 through which wires 110-113 from the electro-magnetic shunt coils extend. Each of grooves 106-109 has a depth greater than the depth of recesses 96, 98 so that the wires will be below the bottoms of recesses 96, 98. Central disc 92 also has transverse grooves in the opposite surfaces thereof aligned with grooves 106-109 for accommodating the lead wires for coils 94. Shunt coil lead wires 110, 111 extend through an elongated tube 114 of ferromagnetic material that is closely received in groove 100. Shunt coil lead wires 112, 113 extend through an elongated tube 115 of ferromagnetic material that is closely received in groove 101.

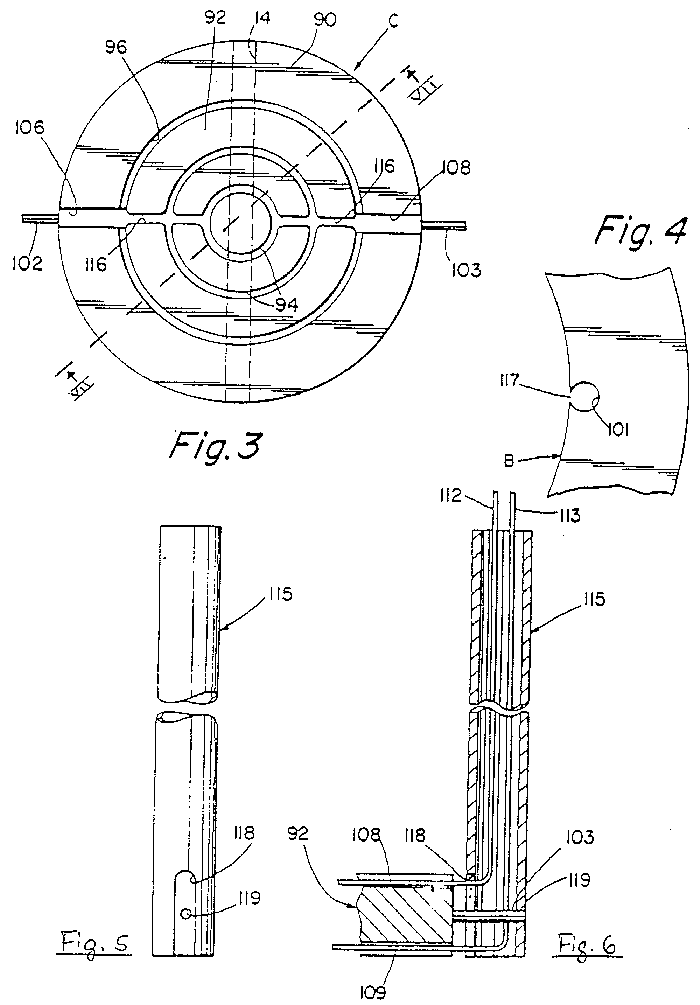

Figure 3 is a plan view of spacer means C with the electro-magnetic shunt coils shown very schematically, because the primary purpose of the figure is to show grooves 106, 108 in ring member 90 and grooves 116 in central disc 92 for accommodating the lead wires for the coils. Figure 3 also shows the guide projections defined by pins 102, 103 as being centrally aligned with grooves 106, 108, and angularly-spaced 90° from hole 14.

Figure 4 shows longitudinal circular groove 101 in housing B as opening at a narrow guide portion 117 along the interior surface of housing B. The width of narrow guide portion 117 of the groove is related to the diameter of a guide pin 103 such that the pin is closely received therein in close sliding relationship thereto.

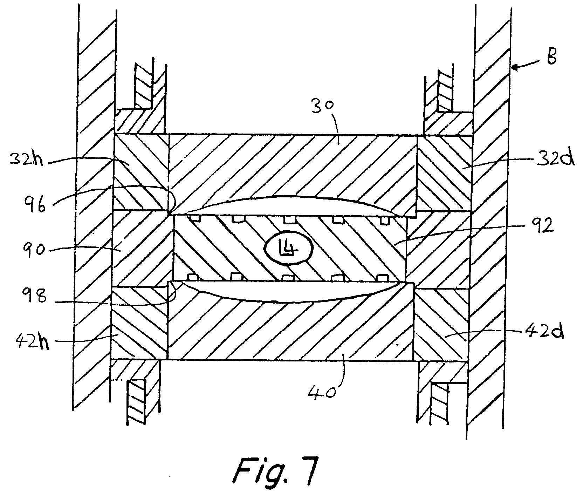

Tube 115 of ferromagnetic material is a close sliding fit in circular groove 101. As shown in Figures 5 and 6, tube 115 has a longitudinal recess 118 in a bottom end portion thereof, and a small transverse hole 119 aligned with recess 118. Pin 103 is closely receivable in hole 119. Groove 100 in housing B, and tube 114 receivable therein, have the same relationship to one another and to pin 102 as described for groove 101, tube 115 and pin 103.

To assemble spacer means C within housing B, the lead wires 110, 111 and 112, 113 are fed through tubes 114, 115, and pins 102, 103 are inserted in the transverse tube holes as shown for pin 103 and hole 119 in Figure 6. The assembled spacer means and tubes are then slidably guided into housing B and grooves 100, 101 until holes 12, 14 are in alignment. A retaining assembly pin can then be inserted into the aligned holes for maintaining spacer means C in position while the other components are assembled within housing B. With grooves 100, 101 located 90° from the axis of housing hole 12, and pins 102, 103 located 90° from the axis of hole 14, the holes are automatically in angular alignment when the spacer means and tubes are slidably guided into the housing and housing grooves.

Heating elements 120, 122 are positioned around sleeves H, I, and are controlled by thermistors for maintaining a desired temperature of the magnet assembly. Flange 72 on sleeve H has an axial groove 124 therein aligned with a hole 126 in cover 52 for extending lead 128 of heating element 120 to the exterior of the housing. Likewise, flange 76 on sleeve I has a groove 130 aligned with a hole 132 in cover 62 for extending lead 134 of heating element 122 to the exterior of the housing.

In the present application, materials having a coercivity substantially the same as air, such as aluminium, brass or plastic, are referred to as non-magnetic or non-ferromagnetic materials for convenience of description. Ferromagnetic materials are those having a very high coercivity, such as iron.

The improvements of the present invention have been shown and described with reference to a cylindrical magnet assembly having a circular cross-sectional shape. However, it will be recognized that the improvements of the present invention can also be used with magnet assemblies having other shapes including, but not necessarily limited to, those having generally square or polygonal cross-sectional shapes.

Although the invention has been shown and described with respect to certain preferred embodiments, it is obvious that equivalent alterations and modifications will occur to others skilled in the art upon the reading and understanding of this specification. The present invention is limited only by the scope of the claims.

## Claims

1. A permanent magnetic assembly for providing a magnetic field across a gap and including opposed magnet members (30,40) having opposite magnetic poles (36,46) facing one another across a gap, and spacer means (C) of non-ferromagnetic material positioned between said magnet members (30,40) for maintaining same in spaced-apart relationship, said magnet members (30,40) having facing end portions (36,46) adjacent said spacer means (C), characterised by cooperating alignment means (96,98) between said magnet members (30,40) and said spacer means (C) for aligning said magnet members along a substantially common longitudinal axis (10) wherein said alignment means comprises opposite shallow recesses (96,98) in said spacer means (C) closely receiving said facing end portions (36,46) of said magnet members (30,40).

2. The magnet assembly of claim 1 including side magnet means (32a-h;42a-h) positioned around said magnet members (30,40) against said spacer means (C) outwardly of said recesses (96,98) therein.

3. The magnet assembly of claim 1 or 2 wherein said end portions of said magnet members (30,40) and said recesses (96,98) are substantially cylindrical.

4. The magnet assembly of claim 1, 2 or 3 wherein said magnet members comprise pole pieces (30,40) having flat ends (34,44) opposite from said facing end portions (36,46), and side magnet means (32a-h;42a-h) positioned around said pole pieces (30,40) and against said spacer means (C) outwardly of said recesses (96,98) therein, said side magnet means having first surfaces positioned against said spacer means (C) and having opposite surfaces that are substantially flush with said flat ends (34,44).

5. The magnet assembly of claim 4 including principal magnets(F,G) having plane ends positioned against said flat ends (34,44) of said pole pieces (30,40), said plane ends having substantially the same shape and slightly larger size than said flat ends (34,44) so that said plane ends overlap a peripheral interface between said pole pieces (30,40) and said side magnets (32a-h;42a-h).

6. A permanent magnet assembly for providing a magnetic field across a gap and including a pair of spaced-apart pole pieces (30,40) having opposed facing end portions (36,46) of opposite magnetic polarity on opposite sides of a gap, said pole pieces having flat ends (34,44) opposite from said facing end portions (36,46), side magnet means (32a-h;42a-h) positioned around said pole pieces (30,40) substantially flush with said flat ends (34,44) of said pole pieces (30,40), said side magnet means (32a-h;42a-h) engaging said pole pieces at a peripheral interface, a pair of main magnets (F,G) having plane ends abutting said flat ends (34,44) of said pole pieces (30,40), said plane ends having the same shape as said flat ends but a slightly larger size such that said plane ends outwardly overlap said peripheral interface, characterised in that each said pole piece (30,40) has a longitudinal axis (10) and an outer periphery and a predetermined thickness adjacent said outer periphery measured parallel to said longitudinal axis (10), said side magnet means (32a-h;42a-h) associated with each said pole piece (30,40) having a thickness measured parallel to said longitudinal axis (10) that is less than said predetermined thickness of its associated pole piece (30,40).

7. The magnet assembly of any preceding claim , further including a ferromagnetic housing (B) having a peripheral wall, opposite ends and an inner peripheral surface, spacer means (C) being provided in said housing for maintaining magnet assemblies (D,E) of opposite magnetic polarity spaced from one another, said spacer means (C) including shunt coils (94) having lead wires (110-113), characterised by at least one longitudinal groove (100,101) in said housing peripheral wall opening outwardly at at least one of said housing ends and being open along the length thereof at said inner peripheral surface, and said lead wires (110-113) extending through said groove (100,101), further including a tube (115) of ferromagnetic material received in said groove (100,101) with a close sliding fit, said lead wires (110,113) extending through said tube (115).

8. The magnet assembly of claim 7 wherein said groove (100,101) opens through said inner peripheral surface along a narrow entrance opening, a guide projection (102,103) extending outwardly from said spacer means (C) for close guiding reception in said narrow entrance opening.

9. The magnet assembly of claim 8 including connecting means for connecting said guide projection (102,103) and said tube (115).

10. The magnet assembly of any of claims 7 to 9 wherein said housing (B) has a pair of said longitudinal grooves (100,101) therein located opposite one another.

11. The magnet assembly of any of claims 7 to 10 wherein said housing (B), said spacer means (C) and said groove (100,101) are all cylindrical.

12. The magnet assembly of any preceding claim, further including a ferromagnetic housing (B) having a peripheral wall, opposite ends and an inner peripheral surface, a lateral housing hole (12) through said peripheral wall, a longitudinal groove (100,101) in said inner peripheral surface of said housing (B) angularly spaced from said housing hole (12) a predetermined angular degree, there being spacer means (C) slidably received in said housing for maintaining magnet assemblies (D,E) of opposite magnetic polarity spaced from one another, said spacer means (C) having a lateral spacer hole (14) therein aligned with said lateral housing hole (12), and a guide projection (102,103) on said spacer means (C) receivable in close guiding relationship in said groove (100,101) and being angularly spaced from said spacer hole (14) substantially the same as said predetermined angular degree, whereby said lateral housing and-spacer holes (12,14) are alignable by sliding said spacer means (C) into said housing (B) with said guide projection (102,103) received in said groove (100,101).

13. The magnet assembly of claim 12 wherein said groove (100,101) has a narrow portion intersecting said inner peripheral surface and has an enlarged portion within said housing periphal wall, said spacer means (C) including an electro-magnetic coil (94) having lead wires (110,113) extending through said enlarged portion of said groove (100,101) to the exterior of said housing (B).

14. The magnet assembly of claim 12, or 13 including a tube (115) of ferromagnetic material closely received in said enlarged portion of said groove (100,101), said lead wires (110-113) extending through said tube (115).

15. The magnet assembly of claim 12, 13 or 14 including connecting means for connecting said guide projection (102,103) with said tube (115).

## Patentansprüche

1. Permanentmagnetanordnung zum Bereitstellen eines Magnetfeldes quer über einen Spalt, umfassend gegenüberliegende Magnetelemente (30, 40) mit entgegengesetzten magnetischen Polen (36, 46), welche sich jeweils über den Spalt hinweg gegenüberliegen, und ein Abstandhaltemittel (C) aus nicht-ferromagnetischem Material, welches zwischen den Magnetelementen (30, 40) angeordnet ist, um diese mit Abstand voneinander angeordnet zu halten, wobei die Magnetelemente (30, 40) dem Abstandhaltemittel (C) benachbarte, aufeinander zuweisende Endbereiche (36, 46) aufweisen, gekennzeichnet durch zwischen den Magnetelementen (30, 40) und dem Abstandhaltemittel (C) angeordnete zusammenwirkende Ausrichtmittel (96, 98), um die Magnetelemente entlang einer im wesentlichen gemeinsamen Längsachse (10) auszurichten, wobei das Ausrichtmittel entgegengesetzte flache Aussparungen (96, 98) in dem Abstandhaltemittel (C) umfaßt, welche die aufeinander zuweisenden Endbereiche (36, 46) der Magnetelemente (30, 40) eng aufnehmen.

2. Magnetanordnung nach Anspruch 1 mit Seitenmagnetmitteln (32a-h; 42a-h), welche um die Magnetelemente (30, 40) herum und außerhalb der Aussparungen (96, 98) in dem Abstandhaltemittel (C) gegen das Abstandhaltemittel (C) angeordnet sind.

3. Magnetanordnung nach Anspruch 1 oder 2, worin die Endbereiche der Magnetelemente (30, 40) und die Aussparungen (96, 98) im wesentlichen zylindrisch sind.

4. Magnetanordnung nach Anspruch 1, 2 oder 3, worin die Magnetelemente Polstücke (30, 40), welche den aufeinander zuweisenden Endbereichen (36, 46) gegenüberliegende ebene Enden (34, 44) aufweisen, sowie Seitenmagnetmittel (32a-h; 42a-h), welche um die Polstücke (30, 40) herum und außerhalb der Aussparungen (96, 98) in dem Abstandhaltemittel (C) gegen das Abstandhaltemittel (C) angeordnet sind, umfassen, wobei die Seitenmagnetmittel gegen das Abstandhaltemittel (C) angeordnete erste Oberflächen und mit den ebene Enden (34, 44) im wesentlichen bündige entgegengesetzte Oberflächen aufweisen.

5. Magnetanordnung nach Anspruch 4 mit Hauptmagneten (F, G), welche plane Enden aufweisen, die gegen die ebene Enden (34, 44) der Polstücke (30, 40) angeordnet sind, wobei die planen Enden im wesentlichen die gleiche Form und eine etwas größere Größe aufweisen, als die ebenen Enden (34, 44), so daß die planen Enden eine Umfangsgrenzfläche zwischen den Polstücken (30, 40) und den Seitenmagneten (32a-h; 42a-h) überdecken.

6. Permanentmagnetanordnung zum Bereitstellen eines magnetischen Feldes quer über einen Spalt, umfassend: ein Paar voneinander mit Abstand angeordneter Polstücke (30, 40), welche an entgegengesetzten Seiten des Spaltes gegenüberliegende, aufeinander zuweisende Endbereiche (36, 46) entgegengesetzter magnetischer Polarität aufweisen, wobei die Polstücke den aufeinander zuweisenden Endbereichen (36, 46) entgegengesetzte ebene Enden (34, 44) aufweisen, sowie Seitenmagnetmittel (32a-h; 42a-h), welche um die Polstücke (30, 40) herum angeordnet und mit den ebenen Enden (34, 44) der Polstücke (30, 40) im wesentlichen bündig sind, wobei die Seitenmagnetmittel (32a-h; 42a-h) an den Polstücken an einer Umfangsgrenzfläche angreifen, sowie ein Paar Hauptmagnete (F, G) mit an den ebenen Enden (34, 44) der Polstücke (30, 40) anliegenden planen Enden, wobei die planen Enden die gleiche Form wie die ebene Enden, jedoch eine etwas größere Größe aufweisen, so daß die planen Enden die Umfangsgrenzfläche nach außen überdecken, dadurch gekennzeichnet, daß jedes Polstück (30, 40) eine Längsachse (10), einen Außenumfang und eine dem Außenumfang benachbarte und parallel zur Längsachse (10) gemessene vorbestimmte Dicke aufweist und die jedem Polstück (30, 40) zugeordneten Seitenmagnetmittel (32a-h; 42a-h) eine parallel zur Längsachse (10) gemessene Dicke aufweisen, welche geringer ist als die vorbestimmte Dicke ihres zugeordneten Polstücks (30, 40).

7. Magnetanordnung nach einem der vorangehenden Ansprüche, welche ferner umfaßt: ein ferromagnetisches Gehäuse (B) mit einer Umfangswandung, entgegengesetzten Enden und einer inneren Umfangsoberfläche, ein Abstandhaltemittel (C), das in dem Gehäuse vorgesehen ist, um Magnetanordnungen (D, E) entgegengesetzter magnetischer Polarität mit Abstand voneinander angeordnet zu halten, wobei das Abstandhaltemittel (C) Nebenspulen (94) mit Anschlußdrähten (110-113) umfaßt, gekennzeichnet durch wenigstens eine Längsausnehmung (100, 101) in der Umfangswandung des Gehäuses, welche sich an wenigstens einem Gehäuseende nach außen öffnet und entlang seiner Länge an der inneren Umfangsoberfläche offen ist, sowie dadurch, daß die Anschlußdrähte (110-113) sich durch die Ausnehmung (100, 101) erstrecken, wobei die Magnetanordnung ferner eine Röhre (115) aus ferromagnetischem Material umfaßt, welche in der Ausnehmung (100, 101) mit einem engem Gleitsitz aufgenommen ist, wobei sich die Anschlußdrähte (110, 113) durch die Röhre (115) erstrecken.

8. Magnetanordnung nach Anspruch 7, worin sich die Ausnehmung (100, 101) durch die innere Umfangsoberfläche entlang einer engen Eingangsöffnung öffnet und sich ein Führungsvorsprung (102, 103) von dem Abstandhaltemittel (C) nach außen erstreckt, um in der engen Eingangsöffnung unter enger Führung aufgenommen zu werden.

9. Magnetanordnung nach Anspruch 8 mit Verbindungsmitteln zum Verbinden des Führungsvorsprungs (102, 103) und der Röhre (115).

10. Magnetanordnung nach einem der Ansprüche 7 bis 9, wobei das Gehäuse (B) ein Paar der Längsausnehmungen (100, 101) in dem Gehäuse (B) aufweist, die entgegengesetzt zueinander angeordnet sind.

11. Magnetanordnung nach einem der Ansprüche 7 bis 10, worin das Gehäuse (B), das Abstandhaltemittel (C) und die Ausnehmung (100, 101) zylindrisch sind.

12. Magnetanordnung nach einem der vorangehenden Ansprüche, ferner umfassend: ein ferromagnetisches Gehäuse (B) mit einer Umfangswandung, entgegengesetzten Enden und einer inneren Umfangsoberfläche, ein in Querrichtung verlaufendes Gehäuseloch (12) durch die Umfangswandung, eine Längsausnehmung (100, 101) in der inneren Umfangsoberfläche des Gehäuses (B), welche von dem Gehäuseloch (12) mit einem vorbestimmten Winkelmaß winkelmäßig beabstandet ist, und ein Abstandhaltemittel (C), welches in dem Gehäuse verschiebbar aufgenommen ist, um Magnetanordnungen (D, E) entgegengesetzter magnetischer Polarität voneinander mit Abstand angeordnet zu halten, wobei das Abstandhaltemittel (C) ein in Querrichtung verlaufendes, zu dem in Querrichtung verlaufenden Gehäuseloch (12) ausgerichtetes Abstandhalterloch (14) aufweist, sowie einen Führungsvorsprung (102, 103) an dem Abstandhaltemittel (C), welcher in der Ausnehmung (100, 101) in enger Führungsbeziehung aufnehmbar ist und von dem Abstandhalterloch (14) im wesentlichen um das gleiche vorbestimmte Winkelmaß winkelmäßig beabstandet ist, wobei die querverlaufenden Löcher (12, 14) im Gehäuse und im Abstandhalter durch Einschieben des Abstandhaltemittels (C) in das Gehäuse (B) bei in der Ausnehmung (100, 101) aufgenommenem Führungsvorsprung (102, 103) ausrichtbar sind.

13. Magnetanordnung nach Anspruch 12, worin die Ausnehmung (100, 101) einen die innere Umfangsoberfläche schneidenden engen Bereich und einen verbreiterten Bereich innerhalb der Gehäuseumfangswandung aufweist, wobei das Abstandhaltemittel (C) eine elektromagnetische Spule (94) mit Anschlußdrähten (110, 113) aufweist, welche sich durch den verbreiterten Bereich der Ausnehmung (100, 101) bis nach außerhalb des Gehäuses (B) erstrecken.

14. Magnetanordnung nach Anspruch 12 oder 13 mit einer Röhre (115) aus ferromagnetischem Material, welche in dem verbreiterten Bereich der Ausnehmung (100, 101) eng aufgenommen ist, wobei sich die Anschlußdrähte (110-113) durch die Röhre (115) erstrecken.

15. Magnetanordnung nach Anspruch 12, 13 oder 14, mit Verbindungsmitteln zum Verbinden des Führungsvorsprungs (102, 103) mit der Röhre (115).

## Revendications

1. Assemblage à aimants permanents pour fournir un champ magnétique à travers un entrefer et présentant des éléments d'aimant opposés (30,40) ayant des pôles (36, 46) magnétiques opposés orientés l'un vers l'autre à travers un entrefer, et un moyen d'écartement (C) en matériau non ferromagnétique placé entre lesdits éléments d'aimant (30, 40) pour maintenir ceux-ci en relation espacée, lesdits éléments d'aimant (30, 40) ayant des parties d'extrémité en vis-à-vis (36, 46) voisines du moyen d'écartement (C), caractérisé par des moyens d'alignement coopérants (96, 98) entre lesdits éléments d'aimant (30, 40) et ledit élément d'écartement (C) pour aligner lesdits éléments d'aimant le long d'un axe longitudinal sensiblement commun (10) dans lequel lesdits moyens d'alignement comprennent des cavités peu profondes opposées (96, 98) dans ledit moyen d'écartement (C) recevant de manière ajustée lesdites parties d'extrémité en vis-à-vis (36, 46) desdits éléments d'aimant (30, 40).

2. Assemblage à aimants selon la revendication 1 présentant des moyens à aimants latéraux (32a à h; 42a à h) placés autour desdits éléments d'aimant (30, 40) contre ledit moyen d'écartement (C) extérieurement auxdites cavités (96, 98) de celui-ci.

3. Assemblage à aimants selon la revendication 1 ou 2, caractérisé en ce que lesdites parties d'extrémité desdits éléments d'aimant (30, 40) et lesdites cavités (96, 98) sont sensiblement cylindriques.

4. Assemblage à aimants selon la revendication 1, 2 ou 3 caractérisé par desdits éléments d'aimant comprenant des pièces polaires (30, 40) ayant des extrémités plates (34, 44) opposées auxdites parties d'extrémité en vis-à-vis (36, 46), et des moyens à aimants latéraux (32a à h; 42a à h) placés autour desdites pièces polaires (30, 40) et contre ledit moyen d'écartement (C) extérieurement auxdites cavités (96, 98), lesdits moyens à aimants latéraux ayant des premières surfaces placées contre ledit moyen d'écartement (C) et ayant des surfaces opposées qui sont sensiblement de niveau avec les extrémités plates (34, 44).

5. Assemblage à aimants selon la revendication 4 présentant des aimants principaux (F, G) ayant des extrémités planes placées contre lesdites extrémités plates (34, 44) desdites pièces polaires (30, 40), lesdites extrémités planes ayant sensiblement la même forme et une taille légèrement plus grande que lesdites extrémités plates (34, 44) de sorte que lesdites extrémités planes recouvrent une interface périphérique entre lesdites pièces polaires (30, 40) et lesdits aimants latéraux (32a à h; 42a à h).

6. Assemblage à aimants permanents pour fournir un champ magnétique à travers un entrefer et présentant deux pièces polaires espacées (30, 40) ayant des parties d'extrémité en vis-à-vis opposées (36, 46) de polarité magnétique opposée sur les côtés opposés d'un entrefer, lesdites pièces polaires ayant des extrémités plates (34, 44) à l'opposé desdites parties d'extrémité en vis-à-vis (36, 46), des moyens à aimants latéraux (32a à h; 42a à h) placés autour desdites pièces polaires (30, 40) sensiblement de niveau avec lesdites extrémités plates (34, 44) desdites pièces polaires (30, 40), lesdits moyens à d'aimants latéraux (32a à h; 42a à h) se mettant en prise avec les pièces polaires à une interface périphérique, une paire aimants principaux (F, G) ayant des extrémités planes butant sur lesdites extrémités plates (34, 44) desdites pièces polaires (30, 40), lesdites extrémités planes ayant la même forme que lesdites extrémités plates mais une taille légèrement supérieure de sorte que lesdites extrémités planes recouvrent vers l'extérieur ladite interface périphérique, caractérisé en ce que chaque pièce polaire (30, 40) a un axe longitudinal (10) et une périphérie extérieure et une épaisseur prédéterminée au voisinage de ladite périphérie extérieure mesurée parallèlement audit axe longitudinal (10), lesdits moyens à aimants latéraux (32a à h; 42a à h) associés à chaque pièce polaire (30, 40) ayant une épaisseur mesurée parallèlement audit axe longitudinal (10) qui est inférieure à ladite épaisseur prédéterminée de sa pièce polaire associée (30, 40).

7. Assemblage à aimants selon l'une quelconque des revendications précédentes, présentant en outre un boîtier ferromagnétique (B) ayant une paroi périphérique, des extrémités opposées et une surface périphérique interne, des moyens d'écartement (C) étant prévus dans ledit boîtier pour maintenir des assemblages d'aimant (D, E) de polarité magnétique opposée espacés l'un par rapport à l'autre, ledit moyen d'écartement (C) présentant des bobines en parallèle (94) ayant des fils conducteurs (110 à 113), caractérisé par au moins une rainure longitudinale (100, 101) dans ladite paroi périphérique du boîtier ouvrant vers l'extérieur à au moins une desdites extrémités et étant ouverte le long de la longueur de celui-ci au niveau de ladite surface périphérique intérieure, et lesdits fils conducteurs (110 à 113) partant à travers ladite rainure (100, 101), présentant en outre un tube (115) en matériau ferromagnétique reçu dans ladite rainure (100, 101) en ajustement glissant juste, lesdits fils conducteurs (110, 113) traversant ledit tube (115).

8. Assemblage à aimants selon la revendication 7, caractérisé en ce que ladite rainure (100, 101) s'ouvre à travers ladite surface périphérique intérieure le long d'une ouverture d'entrée étroite, un prolongement de guidage (102, 103) partant vers l'extérieur depuis ledit moyen d'écartement (C) pour être reçu en guidage ajusté dans ladite ouverture d'entrée étroite.

9. Assemblage à aimants selon la revendication 8 présentant des moyens de connexion pour connecter ledit prolongement de guidage (102, 103) et ledit tube (115).

10. Assemblage à aimants selon l'une quelconque des revendications 7 à 9, caractérisé en ce que ledit boîtier (B) a une paire de rainures longitudinales (100, 101) situées à l'opposé l'une de l'autre.

11. Assemblage à aimants selon l'une quelconque des revendications 7 à 10, caractérisé en ce que ledit boîtier (B), ledit moyen d'écartement (C) et ladite rainure (100, 101) sont tous cylindriques.

12. Assemblage à aimants selon l'une quelconque des revendications précédentes, contenant en outre un boîtier ferromagnétique (B) ayant une paroi périphérique, des extrémités opposées et une surface périphérique intérieure, un orifice de boîtier latéral (12) à travers ladite paroi périphérique, une rainure longitudinale (100, 101) dans ladite surface périphérique intérieure dudit boîtier (B) espacée selon un angle dudit orifice du boîtier (12) d'un degré angulaire prédéterminé, un moyen d'écartement (C) reçu de manière glissante dans ledit boîtier pour maintenir les assemblages à aimants (D, E) de polarité magnétique opposée espacés l'un de l'autre, ledit moyen d'écartement (C) ayant un orifice d'écartement latéral (14) aligné avec ledit orifice latéral du boîtier (12) et un prolongement de guidage (102, 103) sur ledit moyens d'écartement (C) pouvant être reçu en relation de guidage ajusté dans ladite rainure (100, 101) et étant espacé de façon angulaire dudit orifice d'écartement (14) sensiblement du même degré angulaire que ledit degré angulaire prédéterminé, ce qui fait que lesdits orifices latéraux du boîtier et de l'élément d'écartement (12, 14) peuvent être alignés en glissant ledit moyen d'écartement (C) dans ledit boîtier (B), ledit prolongement de guidage (102, 103) étant reçu dans ladite rainure (100, 101).

13. Assemblage à aimants selon la revendication 12 caractérisé en ce que ladite rainure (100, 101) a une partie étroite coupant ladite surface périphérique intérieure et a une partie élargie à l'intérieur de ladite paroi périphérique du boîtier, le moyen d'écartement (C) présentant une bobine électromagnétique (94) ayant des fils conducteurs (110, 113) partant à travers ladite partie élargie de ladite rainure (100, 101) vers l'extérieur dudit boîtier (B).

14. Assemblage à aimants selon la revendication 12, ou 13 présentant un tube (115) en matériau ferromagnétique reçu de manière ajustée dans ladite partie élargie de ladite rainure (100, 101), lesdits fils conducteurs (110 à 113) passant à travers ledit tube (115).

15. Assemblage à aimants selon la revendication 12, 13 ou 14 présentant des moyens de connexion pour connecter ledit prolongement de guidage (102, 103) avec ledit tube (115).
